# EUROPÄISCHE PATENTSCHRIFT

(11) **EP 4 100 083 B1**
(45) Veröffentlichungstag und Bekanntmachung des Hinweises auf die Patenterteilung: **26.04.2023**
(21) Anmeldenummer: 22708105.6
(22) Anmeldetag: 22.02.2022
(51) Int. Cl.: A61M 5/145

(54) **VORRICHTUNG ZUM VERABREICHEN VON FLÜSSIGKEITEN MIT DÄMPFUNGSELEMENT ZUR ABSOPRTION VON EXTERNEN STOSSKRÄFTEN**
DEVICE FOR ADMINISTRATION OF LIQUIDS WITH DAMPING ELEMENT FOR ABSORBING EXTERNAL IMPACT FORCES
DISPOSITIF D'ADMINISTRATION DE LIQUIDES AVEC ELEMENT AMORTISSANT POUR ABSORBER DES FORCES D'IMPACT EXTERNES

(30) Priorität: 24.02.2021 DE 102021104414
(43) Veröffentlichungstag der Anmeldung: 14.12.2022
(73) Patentinhaber: B. Braun Melsungen AG, 34212 Melsungen (DE)
(72) Erfinder: ALJETS, Dirk, 34131 Kassel (DE); ANGERSBACH, Klaus, 34326 Morschen (DE); ERLEN, Christoph, 34132 Kassel (DE); GERLACH, Hans-Josef, 34431 Marsberg (DE)
(74) Vertreter: Winter, Brandl - Partnerschaft mbB
(86) Internationale Anmeldenummer: PCT/EP2022/054344
(87) Internationale Veröffentlichungsnummer: WO 2022/180012

(56) Entgegenhaltungen:
- JP-B2- 4 477 317
- US-A1- 2017 304 538
- US-B2- 8 585 657

## Beschreibung

### Technisches Gebiet

Die vorliegende Offenbarung betrifft eine Vorrichtung zur Verabreichung von medizinischer Flüssigkeit. Insbesondere betrifft die Offenbarung eine Infusionspumpe, vorzugsweise eine Spritzenpumpe.

Infusionspumpen werden im Wesentlichen zwischen Spritzenpumpen und Schlauchpumpen unterschieden. Bei Spritzenpumpen wird eine mit der zu verabreichenden Flüssigkeit gefüllte Spritze durch einen Antrieb bewegt, so dass ein Spritzenkolben der Spritze mit gesteuerter Vorschubgeschwindigkeit bewegt wird, um die Flüssigkeit aus der Spritze heraus zu fördern. Bei Schlauchpumpen wird ein mit der zu verabreichenden Flüssigkeit gefüllter Schlauch durch einen Antrieb, etwa mittels einer Peristaltik, bewegt, um die Flüssigkeit aus dem Schlauch heraus zu fördern.

Eine solche Spritzenpumpe ist beispielsweise aus der WO 2019/213 496 A bekannt. Die Spritzenpumpe weist ein Gehäuse, in dem eine Spritze aufnehmbar ist, und einen von dem Gehäuse seitlich abstehenden Antrieb auf. Eine weitere Spritzenpumpe ist in JP 4 477317 B2 erwähnt.

Bisher bekannte Spritzenpumpen haben bereits einen Stoßschutz, um ihren Antrieb zu schützen. Aufgrund einer steigenden Größe und/oder einem steigenden Gewicht von neu entwickelten Spritzenpumpen, ist die Dämpfung des bisherigen Stoßschutzes nicht ausreichend, so dass der Antrieb, insbesondere ein Antriebskopf des Antriebs, bei einem Fall auf den Antriebskopf beschädigt wird.

Weiterhin ist es bekannt, einen Schutzbügel um gefährdete Bereich der Spritzenpumpe anzubringen, was jedoch den Nachteil hat, dass sich dadurch die Abmessungen der Spritzenpumpe erhöhen, die Spritzenpumpe mit einem Schutzbügel optisch weniger ansprechend sowie aufwändiger zu reinigen ist.

Eine weitere bekannte Lösung zum Schutz der Spritzenpumpe liegt darin, dass die Komponenten der Spritzenpumpe als solche, insbesondere die Komponenten, die in der Kraftkette bei einem Fall auftreten, sehr robust ausgelegt werden, so dass ihnen durch einen Sturz darauf keine Beschädigung zugefügt wird. Eine solche robuste Auslegung macht die Spritzenpumpe als Ganzes jedoch teurer, schwerer und größer, was zu vermeiden ist.

Es ist also Aufgabe der vorliegenden Offenbarung, eine Vorrichtung zur Verabreichung von medizinischer Flüssigkeit, insbesondere eine Spritzenpumpe, bereitzustellen, die eine kompakte Bauweise aufweist und gleichzeitig ihre Funktionalität auch bei einem Sturz gewährleisten kann. Zudem soll die Vorrichtung leicht zu reinigen und kostengünstig in der Herstellung sein.

Die Aufgabe der vorliegenden Offenbarung wird durch eine Vorrichtung mit den Merkmalen des unabhängigen Patentanspruchs 1 gelöst. Vorteilhafte Weiterbildungen sind Gegenstand der Unteransprüche.

Insbesondere ist die Vorrichtung zur Verabreichung von medizinischer Flüssigkeit als eine Spritzenpumpe ausgebildet. Unter einer Spritzenpumpe ist eine Dosierpumpe zu verstehen, mit der die in einer Spritze enthaltene medizinische Flüssigkeit kontinuierlich durch Axialbewegung eines Kolbens der Spritze an einen Patienten abgegeben werden kann. Spritzenpumpen werden oftmals mit ihrem Markennamen als Perfusor bezeichnet.

Die Vorrichtung weist einen Hauptkörper auf. In dem Hauptkörper ist ein Hohlkörper zumindest abschnittsweise aufnehmbar. Der in dem Hauptkörper der Vorrichtung aufnehmbare Hohlkörper enthält die zu verabreichende Flüssigkeit. Im Fall einer Spritzenpumpe ist der Hohlkörper als ein zylindrischer Hohlkörper einer Spritze ausgebildet.

Die Vorrichtung weist einen Antrieb auf. Der Antrieb ist geeignet und ausgebildet, um auf den Hohlkörper derart einzuwirken, dass die Flüssigkeit gefördert wird. Im Fall einer Spritzenpumpe ist der Antrieb insbesondere geeignet, einen Kolben der Spritze axial zu bewegen, so dass die enthaltene Flüssigkeit aus der Spritze herausgedrückt wird. Der Antrieb weist einen von dem Hauptkörper der Vorrichtung abstehenden Antriebskopf auf.

Gemäß der vorliegenden Offenbarung weist die Vorrichtung ein Dämpfungselement auf. Das Dämpfungselement ist zur Absorption von externen Stoßkräften auf den Antriebskopf, insbesondere zur Absorption von kinetischer Energie bei einem Fall/Sturz der Vorrichtung auf den Antriebskopf, auf einer Außenseite des Antriebskopfs angeordnet. Das heißt also, dass das Dämpfungselement außen an dem Antriebskopf angeordnet ist, so dass bei einem Fall/Sturz der Vorrichtung zuerst das Dämpfungselement in Kontakt mit dem Untergrund kommt, bevor die externen Stoßkräfte auf Antriebskopf selbst wirken. So kann die auf den Antriebskopf wirkende Aufprallenergie abgepuffert/verringert werden.

Dies hat den Vorteil, dass der Antriebskopf, der aufgrund seiner von dem Hauptkörper abstehenden Anordnung besonders gefährdet und gleichzeitig für den Antrieb und somit das Fördern der Vorrichtung essentiell ist, zusätzlich geschützt wird.

Gemäß einer bevorzugten Ausführungsform kann der Antriebskopf in axialer Richtung zu dem Hohlkörper (bzw. einem Aufnahmebereich des Hauptkörpers, der zum Aufnehmen des Hohlkörpers geeignet ist) angeordnet sein. Das heißt, dass der Antriebskopf in einer Gebrauchslage der Vorrichtung seitlich von dem Hauptkörper absteht.

Beispielsweise kann der Antrieb als ein als ein Linearantrieb ausgebildet sein. Insbesondere kann der Linearantrieb einen Antriebsmotor und eine mit dem Antriebsmotor gekoppelte Spindel aufweisen. Ein solcher Linearantrieb verändert seine Axialposition und steht insbesondere bei einer vollständig gefüllten Spritze weit von dem Hauptkörper der Vorrichtung ab, so dass er besonders zu schützen ist.

Gemäß einer bevorzugten Ausführungsform kann das Dämpfungselement an einer Stirnseite des von dem Hauptkörper abstehenden Antriebskopfs vorgesehen sein, um Stoßkräfte in axialer Richtung auf den Antriebskopf zu absorbieren. Dies hat den Vorteil, dass die Stoßkräfte besonders geeignet bei einem Fall/Sturz entlang der Axialrichtung, d.h. bei einem senkrechten Fall, auf den Untergrund abgefangen werden können.

Gemäß einer anderen bevorzugten Ausführungsform kann das Dämpfungselement den von dem Hauptkörper abstehenden Antriebskopf zumindest teilweise umhüllen, um Stoßkräfte in radialer und axialer Richtung auf den Antriebskopf zu absorbieren. Durch die umhüllende Anordnung können auch die Sturzkräfte abgefangen werden, die entstehen, wenn die Vorrichtung in einer geneigten Lage auf den Untergrund auftrifft. So ist der Antriebskopf zu jedem Zeitpunkt geschützt.

Gemäß einer bevorzugten Ausführungsform kann das Dämpfungselement aus einem auf Polyurethan basierenden Gel, insbesondere aus einem Technogel, aufgebaut sein. Ein solches Gel hat besonders gute Stoßdämpfungseigenschaften und ist demnach für die Verwendung als Stoßdämpfer geeignet.

Gemäß der bevorzugten Ausführungsform kann das Dämpfungselement derart ausgebildet sein, dass sich das Gel zur Absorption von kinetischer Energie bei einem Fall der Vorrichtung auf den Antriebskopf verformt. Die Verformung des Gels kann eine elastische Verformung sein, was den Vorteil hat, dass die Vorrichtung nach einem Fall weiterverwendet werden kann, ohne das Gel austauschen zu müssen. Die Verformung des Gels kann auch eine plastische Verformung sein, was den Vorteil hat, dass höhere Stoßkräfte aufgenommen werden können. Die Verformung des Gels kann auch teilweise elastisch und teilweise plastisch sein, so dass die Vorteile kombiniert werden, und da Gel beispielsweise bei leichten Stößen weiterverwendbar und bei großen Stößen ausgetauscht werden muss.

Gemäß der bevorzugten Ausführungsform kann das Gel eine Wabenstruktur besitzen, wobei die Waben vorzugsweise in Richtung von dem Antriebskopf weg abstehen. Eine Wabenstruktur ist eine geeignete Möglichkeit, um die Verformung des Gels zu ermöglichen.

Gemäß einer anderen bevorzugten Ausführungsform kann das Dämpfungselement aus Silikon aufgebaut sein. Silikondämpfer haben sich sowohl als besonders effektiv für die Stoßdämpfung als auch als kostengünstig herstellbar erwiesen.

Gemäß der anderen bevorzugten Ausführungsform kann das Dämpfungselement derart ausgebildet sein, dass es zur Absorption von kinetischer Energie bei einem Fall der Vorrichtung auf den Antriebskopf den Luftdruck von in dem Silikon eingeschlossener Luft verändert. Das heißt also, dass die kinetische Energie zumindest teilweise in Druckenergie umgewandelt werden kann.

Gemäß der anderen bevorzugten Ausführungsform kann das Dämpfungselement aus Silikon eine Wabenstruktur besitzen, wobei die Waben vorzugsweise in Richtung von dem Antriebskopf weg abstehen. Eine Wabenstruktur ist eine geeignete Möglichkeit, um die Wandlung der kinetischen Energie in Druckenergie der Luft, die in den einzelnen Waben eingeschlossen ist, zu ermöglichen.

Gemäß einer bevorzugten Ausführungsform kann die Vorrichtung eine Kappe und/oder eine Abdeckung aufweisen, die das Dämpfungselement nach außen hin bedeckt und/oder das Dämpfungselement an dem Antriebskopf befestigt. Beispielsweise kann die Kappe aus Kunststoff ausgebildet sein, da sie sich so besonders einfach reinigen lässt. Durch die Kappe, unter der das Dämpfungselement angeordnet ist, kann eine einfache Befestigung des Dämpfungselements an dem Antriebskopf gewährleistet werden. Vorzugsweise kann das Dämpfungselement, beispielsweise durch die Kappe, lösbar an dem Antriebskopf angebracht sein. So kann das Dämpfungselement bei Bedarf ausgetauscht werden.

Gemäß der bevorzugten Ausführungsform kann die Kappe vorzugsweise durch einen elastischen Formschluss, wie eine Schnappverbindung, an dem Antriebskopf angebracht sein. Das heißt, dass die Kappe verbindungselementlos, insbesondere direkt, an dem Antriebskopf angebracht ist, so dass keine Verbindungselemente abstehen, an denen sich Verunreinigungen sammeln könnten. In einer Ausführungsform kann der elastische Formschluss durch eine Vertiefung an der Außenseite des Antriebskopfs, in die ein Vorsprung an einer Innenseite der Kappe eingreift, ausgebildet sein. Alternativ kann der elastische Formschluss durch eine Vertiefung in der Innenseite der Kappe, in die ein Vorsprung an der Außenseite der Vorrichtung eingreift, ausgebildet sein.

Gemäß einer bevorzugten Ausführungsform kann das Dämpfungselement in Abhängigkeit einer bei üblicher Verwendung maximal zu erwartenden Fallhöhe, insbesondere 1,80 m, und/oder eines Gewichts der Vorrichtung dimensioniert sein. So kann gewährleistet werden, dass die Vorrichtung in Abhängigkeit ihrer Verwendung bei einem Sturz nicht beschädigt wird.

Mit anderen Worten betrifft die vorliegende Offenbarung eine Vorrichtung zur Verabreichung von medizinischer Flüssigkeit, insbesondere eine Spritzenpumpe, bei der der Antriebskopf zum Schutz mit einem Dämpfungselement ausgestattet ist. Dieses Dämpfungselement setzt die Kräfte, welche bei einem Sturz der Vorrichtung auf einen harten Untergrund auf die Vorrichtung und dessen Komponenten wirkt, auf ein unkritisches Maß herab. Der Stoßschutz im Antriebskopf ist dabei derart ausgewählt, dass die im Falle eines Sturzes freigesetzte potentielle Energie in die Verformung des Dämpfungselements bzw. in die Umwandlung in Druckenergie von in dem Dämpfungselement eingeschlossener Luft umgesetzt wird. Somit kann der Antriebskopf auch bei einem Fall direkt auf den Antriebskopf weiter fördern, und innenliegende Bauteile der Vorrichtung werden nicht beschädigt. Vorzugsweise wird der Stoßschutz der Spritzenpumpe durch eine Kappe/Stoßschutzkappe und ein unter der Kappe angeordnetes Dämpfungselement aus einem Technogel, d.h. einem auf Polyurethan basierenden Gel, oder eine unter der Kappe angeordnete Wabenstruktur aus Silikon gebildet. Dabei dämpft der Stoßschutz einen Fall/Sturz derart, dass der Antrieb beim Fall auf den Antriebskopf nicht beschädigt wird. Zusammenfassen hat die Spritzenpumpe ein unter einer Kunststoffkappe sitzendes, zusätzliches Dämpfungselement, das einen Stoß mildert, so dass bei einem Fall auf den Antriebsfall der Antrieb auch dann funktionsfähig bleibt, wenn das Vorrichtungsgewicht steigt und der Antrieb beim Fall höheren Kräften ausgesetzt ist.

### Kurzbeschreibung der Figuren

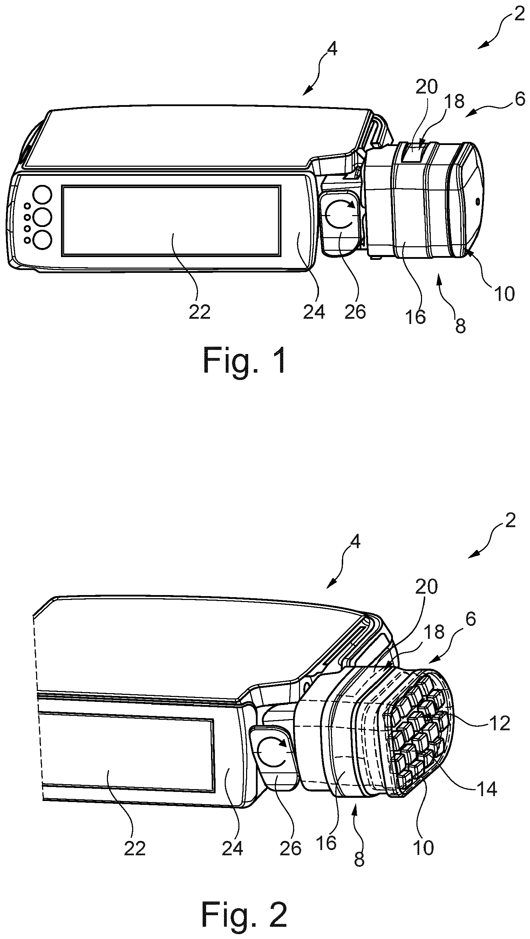

Fign. 1 und 2 sind perspektivische Darstellung einer Vorrichtung gemäß der vorliegenden Offenbarung mit einem Dämpfungselement.

### Beschreibung einer bevorzugten Ausführungsform

Nachstehend wird eine bevorzugte Ausführungsform der vorliegenden Offenbarung auf der Basis der zugehörigen Figuren beschrieben.

Fign. 1 und 2 zeigen eine Vorrichtung 2 zur Verabreichung von medizinischer Flüssigkeit. Die Vorrichtung 2 kann insbesondere als eine Spritzenpumpe ausgebildet sein. Die Vorrichtung 2 weist einen Hauptkörper 4 auf. In dem Hauptkörper 4 ist ein Hohlkörper (nicht dargestellt) zumindest abschnittsweise aufnehmbar. Der in dem Hauptkörper 4 der Vorrichtung 2 aufnehmbare Hohlkörper enthält die zu verabreichende Flüssigkeit. Im Fall einer Spritzenpumpe ist der Hohlkörper als ein zylindrischer Hohlkörper einer Spritze ausgebildet.

Die Vorrichtung 2 weist einen Antrieb 6 auf. Der Antrieb 6 ist geeignet und ausgebildet, um auf den Hohlkörper derart einzuwirken, dass die Flüssigkeit gefördert wird. Im Fall einer Spritzenpumpe ist der Antrieb 6 insbesondere geeignet, einen Kolben der Spritze axial zu bewegen, so dass die enthaltene Flüssigkeit aus der Spritze herausgedrückt wird. Der Antrieb 6 weist einen von dem Hauptkörper 4 der Vorrichtung 2 abstehenden Antriebskopf 8 auf. Der Antriebs 6 ist vorzugsweise als ein Linearantrieb ausgebildet. Der Antriebskopf 8 kann in axialer Richtung zu dem Hohlkörper angeordnet sein. Das heißt, dass der Antriebskopf 8 in einer Gebrauchslage der Vorrichtung 2 seitlich von dem Hauptkörper 4 absteht.

Die Vorrichtung 2 weist ein Dämpfungselement 10 auf. Das Dämpfungselement 10 ist zur Absorption von externen Stoßkräften auf den Antriebskopf 8 auf einer Außenseite des Antriebskopfs 8 angeordnet. Insbesondere dient das Dämpfungselement 10 zur Absorption von kinetischer Energie bei einem Fall/Sturz der Vorrichtung 2 auf den Antriebskopf 8. Vorzugsweise kann das Dämpfungselement 10 lösbar an dem Antriebskopf 8 angebracht sein.

Gemäß einer bevorzugten Ausführungsform kann das Dämpfungselement 10 in Abhängigkeit einer bei üblicher Verwendung maximal zu erwartenden Fallhöhe, insbesondere 1,80 m, und/oder eines Gewichts der Vorrichtung dimensioniert sein.

Vorzugsweise kann das Dämpfungselement 10 an einer Stirnseite des von dem Hauptkörper 4 abstehenden Antriebskopfs 8 vorgesehen sein, um Stoßkräfte in axialer Richtung auf den Antriebskopf 8 zu absorbieren. Alternativ kann das Dämpfungselement 10 den von dem Hauptkörper abstehenden Antriebskopf 8 zumindest teilweise umhüllen, um Stoßkräfte in radialer und axialer Richtung auf den Antriebskopf zu absorbieren.

Das Dämpfungselement 10 kann insbesondere aus einem auf Polyurethan basierenden Gel, insbesondere aus einem Technogel, aufgebaut sein. Vorzugsweise kann das Dämpfungselement 10 derart ausgebildet sein, dass sich das Gel zur Absorption von kinetischer Energie bei einem Fall der Vorrichtung 2 auf den Antriebskopf 8 plastisch und/oder elastisch verformt.

Alternativ kann das Dämpfungselement 10 aus Silikon aufgebaut sein. Vorzugsweise kann das Dämpfungselement 10 derart ausgebildet sein, dass es zur Absorption von kinetischer Energie bei einem Fall der Vorrichtung 2 auf den Antriebskopf 8 den Luftdruck von in dem Silikon eingeschlossener Luft verändert. Das heißt also, dass die kinetische Energie zumindest teilweise in Druckenergie umgewandelt werden kann.

Insbesondere in Fig. 2 ist zu erkennen, dass das Dämpfungselement 10 eine Wabenstruktur besitzen kann. Das heißt, dass das Dämpfungselement 10 wabenartige Vorsprünge 12 besitzt, die voneinander durch Spalte 14 zwischen den einzelnen wabenartigen Vorsprüngen 12 getrennt sind. Vorzugsweise stehen die wabenartigen Vorsprüngen 12 in Richtung, beispielsweise in Axialrichtung, von dem Antriebskopf weg ab.

Zudem kann die Vorrichtung 2 eine Kappe (/eine Abdeckung) 16 aufweisen. Die Kappe 16 ist in Fig. 2 transparent dargestellt, so dass die unter der Kappe 16 angeordneten Bauteile, insbesondere das Dämpfungselement 10, zu erkennen ist.

Die Kappe 16 kann das Dämpfungselement 10 nach außen hin bedecken und/oder das Dämpfungselement 10 an der Vorrichtung 2, insbesondere an dem Antriebskopf 8, befestigen. Vorzugsweise ist das Dämpfungselement 10 vollständig unter der Kappe 16 angeordnet. So wird insbesondere vermieden, dass sich Verunreinigungen an dem Dämpfungselement 10, insbesondere an der Wabenstruktur, festsetzen. Die Kappe 16 kann als eine Kunststoffkappe ausgebildet sein.

Die Kappe 16 kann das Dämpfungselement 10 lösbar an dem Antriebskopf 8 befestigen. Vorzugsweise kann die Kappe 16 durch einen elastischen Formschluss, wie eine Schnappverbindung, an dem Antriebskopf 8 angebracht sein. Das heißt, dass die Kappe 16 verbindungselementlos, insbesondere direkt, an dem Antriebskopf 8 angebracht sein kann. In der dargestellten Ausführungsform kann der elastische Formschluss durch eine Vertiefung 18 an der Außenseite der Vorrichtung 2 (des Antriebskopfs 8), in die ein Vorsprung 20 an einer Innenseite der Kappe 16 eingreift, ausgebildet sein. Auch wenn dies nicht dargestellt ist, kann der elastische Formschluss alternativ durch eine Vertiefung in der Innenseite der Kappe 16, in die ein Vorsprung an der Außenseite der Vorrichtung 2 (des Antriebskopfs 8) eingreift, ausgebildet sein.

Zusätzlich weist die Vorrichtung 2 eine Anzeigeeinheit 22 zur graphischen Darstellung von betriebsrelevanten Parametern der Vorrichtung 2 auf. Die Anzeigeeinheit 22 ist in einer Abdeckung 24 integriert, die durch Betätigung eines Hebels 26 geöffnet werden kann, um den Hohlkörper in den Hauptkörper 4 der Vorrichtung 2 einzulegen und den Hohlkörper aus dem Hauptkörper 4 der Vorrichtung zu entnehmen.

## Patentansprüche

1. Vorrichtung (2) zur Verabreichung von medizinischer Flüssigkeit, insbesondere Spritzenpumpe, mit:
einem Hauptkörper (4), in dem ein Hohlkörper, der die zu verabreichende Flüssigkeit enthält, zumindest abschnittsweise aufnehmbar ist; und
einem Antrieb (6), der geeignet und ausgebildet ist, um auf den Hohlkörper derart einzuwirken, dass die Flüssigkeit gefördert wird, wobei der Antrieb (6) einen von dem Hauptkörper (4) abstehenden Antriebskopf (8) aufweist,
**gekennzeichnet durch**
ein Dämpfungselement (10), das zur Absorption von externen Stoßkräften auf den Antriebskopf (8), insbesondere zur Absorption von kinetischer Energie bei einem Fall der Vorrichtung (2) auf den Antriebskopf (8), auf einer Außenseite des Antriebskopfs (8) angeordnet ist.

2. Vorrichtung (2) nach Anspruch 1, **dadurch gekennzeichnet, dass** das Dämpfungselement (10) an einer Stirnseite des von dem Hauptkörper (4) abstehenden Antriebskopfs (8) vorgesehen ist, um Stoßkräfte in axialer Richtung auf den Antriebskopf (8) zu absorbieren.

3. Vorrichtung (2) nach Anspruch 1, **dadurch gekennzeichnet, dass** das Dämpfungselement (10) den von dem Hauptkörper (4) abstehenden Antriebskopf (8) zumindest teilweise umhüllt, um Stoßkräfte in radialer und axialer Richtung auf den Antriebskopf (8) zu absorbieren.

4. Vorrichtung (2) nach einem der Ansprüche 1 bis 3, **dadurch gekennzeichnet, dass** das Dämpfungselement (10) aus einem auf Polyurethan basierenden Gel, insbesondere Technogel, aufgebaut ist.

5. Vorrichtung (2) nach Anspruch 4, **dadurch gekennzeichnet, dass** das Dämpfungselement (10) derart ausgebildet ist, dass sich das Gel zur Absorption von kinetischer Energie bei einem Fall der Vorrichtung (2) auf den Antriebskopf (8) verformt.

6. Vorrichtung (2) nach einem der Ansprüche 1 bis 3, **dadurch gekennzeichnet, dass** das Dämpfungselement (10) aus Silikon aufgebaut ist.

7. Vorrichtung (2) nach Anspruch 6, **dadurch gekennzeichnet, dass** das Dämpfungselement (10) derart ausgebildet ist, dass es zur Absorption von kinetischer Energie bei einem Fall der Vorrichtung (2) auf den Antriebskopf (8) den Luftdruck von in dem Dämpfungselement (10) aus Silikon eingeschlossener Luft verändert.

8. Vorrichtung (2) nach einem der Ansprüche 4 bis 7, **dadurch gekennzeichnet, dass** das Dämpfungselement (10) eine Wabenstruktur besitzt, wobei die Waben vorzugsweise in Richtung von dem Antriebskopf (8) weg abstehen.

9. Vorrichtung (2) nach einem der Ansprüche 1 bis 8, **gekennzeichnet durch** eine vorzugsweise aus Kunststoff ausgebildete Kappe (16), die das Dämpfungselement (10) nach außen hin bedeckt und/oder das Dämpfungselement (10) an dem Antriebskopf (8), insbesondere lösbar, befestigt, wobei die Kappe (16) vorzugsweise durch einen elastischen Formschluss an dem Antriebskopf (8) angebracht ist.

10. Vorrichtung (2) nach einem der Ansprüche 1 bis 9, **dadurch gekennzeichnet, dass** das Dämpfungselement (10) in Abhängigkeit einer bei üblicher Verwendung maximal zu erwartenden Fallhöhe, insbesondere 1,80 m, und/oder eines Gewichts der Vorrichtung (2) dimensioniert ist.

## Claims

1. A device (2) for administering a medical liquid, in particular a syringe pump, comprising:
a main part (4) in which a hollow body containing the liquid to be administered can be at least partly received; and
a drive (6) which is suitable and designed for acting on the hollow body such that the liquid is conveyed, wherein the drive (6) includes a drive head (8) projecting from the main body (4),
**characterized by**
a damping element (10) which is disposed on an outside of the drive head (8) for absorbing external impact forces acting on the drive head (8), in particular for absorbing kinetic energy, if the device (2) falls onto the drive head (8).

2. The device (2) according to claim 1, **characterized in that** the damping element (10) is provided on an end side of the drive head (8) projecting from the main part (4) to absorb impact forces acting in an axial direction on the drive head (8).

3. The device (2) according to claim 1, **characterized in that** the damping element (10) at least partly envelops the drive head (8) projecting from the main part (4) to absorb impact forces acting in radial and axial directions on the drive head (8).

4. The device (2) according to any one of the claims 1 to 3, **characterized in that** the damping element (10) is made of a polyurethane-based gel, specifically a technogel.

5. The device (2) according to claim 4, **characterized in that** the damping element (10) is designed such that the gel deforms for absorbing kinetic energy if the device (2) falls onto the drive head (8).

6. The device (2) according to any one of the claims 1 to 3, **characterized in that** the damping element (10) is made of silicone.

7. The device (2) according to claim 6, **characterized in that** the damping element (10) is designed such that it alters the air pressure of air enclosed in the damping element (10) made of silicone for absorbing kinetic energy if the device (2) falls onto the drive head (8).

8. The device (2) according to any one of the claims 4 to 7, **characterized in that** the damping element (10) has a honeycomb structure, the honeycombs projecting preferably in a direction away from the drive head (8).

9. The device (2) according to any one of the claims 1 to 8, **characterized by** a cap (16) preferably made of plastic which covers the damping element (10) to the outside and/or secures the damping element (10), specifically detachably, to the drive head (8), the cap (16) being arranged on the drive head (8) preferably by an elastic form closure.

10. The device (2) according to any one of the claims 1 to 9, **characterized in that** the damping element (10) is dimensioned depending on a maximum fall height to be expected during normal use, specifically 1.80 m, and/or on a weight of the device (2).

## Revendications

1. Dispositif (2) pour l'administration de liquide médical, en particulier pompe à seringue, avec :
un corps principal (4), dans lequel un corps creux qui contient le liquide à administrer, peut être reçu au moins par sections ; et
un entraînement (6) qui est approprié et réalisé afin d'agir sur le corps creux de telle manière que le liquide soit refoulé, dans lequel l'entraînement (6) présente une tête d'entraînement (8) dépassant du corps principal (4),
**caractérisé par**
un élément d'amortissement (10) qui est agencé pour l'absorption de forces d'impact externes sur la tête d'entraînement (8), en particulier pour l'absorption d'énergie cinétique en cas de chute du dispositif (2) sur la tête d'entraînement (8), sur un côté extérieur de la tête d'entraînement (8).

2. Dispositif (2) selon la revendication 1, **caractérisé en ce que** l'élément d'amortissement (10) est prévu au niveau d'un côté frontal de la tête d'entraînement (8) dépassant du corps principal (4) afin d'absorber des forces d'impact dans le sens axial sur la tête d'entraînement (8).

3. Dispositif (2) selon la revendication 1, **caractérisé en ce que** l'élément d'amortissement (10) enveloppe au moins partiellement la tête d'entraînement (8) dépassant du corps principal (4) afin d'absorber des forces d'impact dans le sens radial et axial sur la tête d'entraînement (8).

4. Dispositif (2) selon l'une quelconque des revendications 1 à 3, **caractérisé en ce que** l'élément d'amortissement (10) est constitué d'un gel à base de polyuréthane, en particulier de Technogel.

5. Dispositif (2) selon la revendication 4, **caractérisé en ce que** l'élément d'amortissement (10) est réalisé de telle manière que le gel se déforme pour l'absorption d'énergie cinétique en cas de chute du dispositif (2) sur la tête d'entraînement (8).

6. Dispositif (2) selon l'une quelconque des revendications 1 à 3, **caractérisé en ce que** l'élément d'amortissement (10) est constitué de silicone.

7. Dispositif (2) selon la revendication 6, **caractérisé en ce que** l'élément d'amortissement (10) est réalisé de telle manière qu'il modifie la pression de l'air inclus dans l'élément d'amortissement (10) en silicone pour l'absorption d'énergie cinétique en cas de chute du dispositif (2) sur la tête d'entraînement (8).

8. Dispositif (2) selon l'une quelconque des revendications 4 à 7, **caractérisé en ce que** l'élément d'amortissement (10) possède une structure en nid d'abeille, dans lequel les alvéoles dépassent de préférence dans une direction éloignée de la tête d'entraînement (8).

9. Dispositif (2) selon l'une quelconque des revendications 1 à 8, **caractérisé par** un capuchon (16) réalisé de préférence en matière plastique qui recouvre l'élément d'amortissement (10) vers l'extérieur et/ou fixe l'élément d'amortissement (10) à la tête d'entraînement (8), en particulier de manière amovible, dans lequel le capuchon (16) est monté de préférence par une liaison à complémentarité de formes élastique au niveau de la tête d'entraînement (8).

10. Dispositif (2) selon l'une quelconque des revendications 1 à 9, **caractérisé en ce que** l'élément d'amortissement (10) est dimensionné en fonction d'une hauteur de chute à attendre lors de l'utilisation usuelle au maximum, en particulier 1,80 m, et/ou d'un poids du dispositif (2).
